# EUROPÄISCHE PATENTSCHRIFT

(11) **EP 1 139 760 B1**
(45) Veröffentlichungstag und Bekanntmachung des Hinweises auf die Patenterteilung: **14.05.2003**
(21) Anmeldenummer: 99963540.2
(22) Anmeldetag: 15.12.1999
(51) Int. Cl.: A01N 57/20, A01N 43/16, C07D 309/20

(54) **CYCLOHEXENONOXIMETHER / (GLYPHOSATE / GLUPHOSINATE) - SUSPENSIONSKONZENTRATE**
CYCLOHEXENONE OXIME ETHER / (GLYPHOSATES / GLUPHOSINATES) SUSPENSION CONCENTRATES
CONCENTRES EN SUSPENSION DE CYCLOHEXENONOXIMETHER / (GLYPHOSATE / GLUPHOSINATE)

(30) Priorität: 15.12.1998 US 211011
(43) Veröffentlichungstag der Anmeldung: 10.10.2001
(73) Patentinhaber: BASF AKTIENGESELLSCHAFT, 67056 Ludwigshafen (DE)
(72) Erfinder: VON DER HEYDE, Jürgen, D-64625 Bensheim (DE); KOBER, Reiner, D-67136 Fu gönheim (DE); BRATZ, Matthias, D-67117 Limburgerhof (DE); BERGHAUS, Rainer, D-67346 Speyer (DE); JÄGER, Karl-Friedrich, D-67117 Limburgerhof (DE); FRIES, Jürgen, D-67067 Ludwigshafen (DE); PARG, Adolf, D-67098 Bad Dürkheim (DE)
(74) Vertreter: Kinzebach, Werner, Dr.
(86) Internationale Anmeldenummer: EP9909956
(87) Internationale Veröffentlichungsnummer: WO00035288

(56) Entgegenhaltungen:
- EP-A- 0 071 973
- EP-A- 0 394 847
- EP-A- 0 691 127
- WO-A-00/08937
- WO-A-96/29869
- WO-A-98/00008
- DE-A- 19 701 123
- US-A- 4 931 080
- DATABASE WPI Section Ch, Week 198722 Derwent Publications Ltd., London, GB; Class C01, AN 1987-153209 XP002136491 & JP 62 089653 A (NIPPON SODA CO), 24. April 1987 (1987-04-24)

## Beschreibung

Die vorliegende Erfindung betrifft neue, weitgehend wasserfreie Formulierung von Pflanzenschutzwirkstoffen, bestehend im Wesentlichen aus
a) einem Cyclohexenonoximether der Formel I 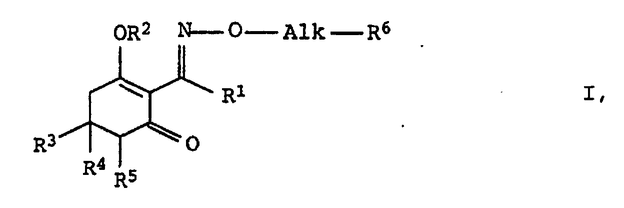 wobei die Variablen folgende Bedeutungen haben:
   - R¹: Ethyl oder Propyl;
   - R²: Wasserstoff oder ein Äquivalent eines landwirtschaftlich brauchbaren Kations;
   - R³: 2-(Thioethyl)propyl, Tetrahydrothiopyran-3-yl, Tetrahydrothiopyran-4-yl, Tetrahydropyran-3-yl, Tetrahydropyran-4-yl, 1-(Methylthio)cyclopropyl, 5-(Isopropyl)isoxazol-3-yl, 2,5-Dimethylpyrazol-3-yl, 2,4,6-Trimethylphenyl oder 2,4,6-Trimethyl-3-butyrylphenyl;
   - R⁴ und R⁵: unabhängig voneinander
   Wasserstoff, Methyl oder Methoxycarbonyl;
   - Alk: CH₂CH₂, CH₂CH(CH₃), CH₂CH=CH, CH₂CH=C(Cl) oder CH₂CH₂CH=CH;
   - R⁶: Wasserstoff, Phenyl, Halogenphenyl, Dihalogenphenyl, Phenoxy, Halogenphenoxy oder Dihalogenphenoxy;
b) N-Phosphonomethylglycin (Glyphosate), ein Ester oder Salz hiervon, DL-Homoalanin-4-yl(methyl)phosphinsäure (Gluphosinate) oder dessen Ammoniumsalz;
c) 20 bis 80 Gew.% eines aprotischen oder schwach protischen Lösungsmittels, in dem die Komponenten a) und b) gelöst oder suspendiert sind;
d) gewünschtenfalls Emulgatoren, Tenside, oberflächenaktive und/oder wirkungssteigernde Hilfsmittel.

Außerdem betrifft die Erfindung Verfahren zur Herstellung einer Spritzbrühe zur Bekämpfung von unerwünschten Pflanzen.

Bekanntermaßen neigen Herbizide aus der Stoffklasse der Cyclohexenonoximethern in wäßrigen oder protischen Solventien zu hydrolytischer Zersetzung oder chemischen Abbaureaktionen. Aus diesem Grund wurden Cyclohexenonoximether bisher lediglich als wasserfreie Emulsionskonzentrate aufbereitet und eingesetzt.

Eine mögliche Alternative bieten Feststoffformulierungen, wie sie in der WO 96/29869 beschrieben wurden.

Feststoff-Formulierungen haben jedoch den Nachteil, daß wirkungssteigernde Zusätze - z.B. lipophile Ester wie Methyloleat, Laurinsäure- und Adipinsäure-Ester, sowie Paraffinöle oder Fettsäure-Ester, gegebenenfalls in Kombination mit ausgewählten Emulgatoren - entweder gar nicht oder nur in sehr geringen Mengen eingearbeitet werden können.

Andererseits wäre es mit Mischungen aus den Cyclohexenonoximethern I und Glyphosate, Gluphosinate oder einem Derivat hiervon möglich, unerwünschte Gräser besser zu bekämpfen als mit Glyphosate/Gluphosinate allein. Eine Bekämpfung von "Round up Ready"-Ausfallmais in "Round up Ready"-Soja wird dadurch sogar erst wirtschaftlich möglich. Dies gilt allgemein für Gräser, die gegenüber Glyphosate resistent (tolerant) sind.

Aufgabe der vorliegenden Erfindung war es deshalb, eine flüssige Fertigformulierung zur Verfügung zu stellen, die ein Cyclohexenonoximether-Herbizid, N-Phosphonomethylglycin oder ein Derivat hiervon und gewünschtenfalls ein wirkungssteigerndes Hilfsmittel enthält und dabei ausreichend lagerstabil ist.

Demgemäß wurden die eingangs definierten Suspensionskonzentrate gefunden. Ferner wurde ein Verfahren zur Herstellung einer herbizid wirksamen Spritzbrühe gefunden.

Kennzeichnend für die vorliegenden Suspensionskonzentrate ist, daß der Anteil an freiem Wasser (das nicht als Kristallwasser gebunden ist) 0 bis 5 Gew.%, bevorzugt 0 bis 2 Gew.%, insbesondere nur 0 bis 0,5 Gew.%, beträgt.

Als Herbizid-Komponenete a) kommen vorzugsweise Cyclohexenonoximether aus der Gruppe: Sethoxydim, Cycloxydim, Clethodim, Tralkoxydim, Butroxydim, 2-[1-(3-Chlorallyloxy)iminopropyl]-5-(tetrahydropyran-4-yl) -3-hydroxy-cyclohex-2-enon, 2-[1-(2-p-Chlorphenoxypropyloxy)-iminobutyl]-5-(tetrahydrothiopyran-3-yl)-3-hydroxycyclohex-2-enon oder deren Gemische in Betracht, insbesondere Clethodim oder 2-[1-(3-Chlorallyloxy)iminopropyl]-5-(tetrahydropyran-4-yl)-3-hydroxy-cyclohex-2-enon. Diese Wirkstoffe sind üblicherweise in der Ölphase löslich. Geeignet sind jedoch auch die Alkalimetall- oder Erdalkalimetall-Salze der vorzugsweise oder insbesondere genannten Cyclohexenonoximether, die in Kombination mit ausgewählten Hilfsmitteln in der kontinuierlichen Ölphase weitgehend unlöslich sind, wodurch der Abbau der Wirkstoffe I weitgehend verhindert wird. So sind diejenigen Cyclohexenanoxinether-Salze bevorzugt, die in der Ölphase < 1 % gelöst, ganz besonders bevorzugt < 0,1 - 0,5 %, vorliegen.

Die Salze der Cyclohexenonoximether I sind in der Regel durch Umsetzung der freien Verbindungen I (R² = Wasserstoff) mit basischen Metallsalz-Lösungen erhältlich. Geeignete basische Metallsalz-Quellen sind dabei typischerweise Hydroxide, Carbonate oder Phospate von Alkali-, Erdalakali oder Übergangsmetallen. Höherwertige Kationen wie Calcium und Magnesium, die ca. äquimolar zu den Verbindungen I eingesetzt wurden, können zusätzlich zur Ladungsabsättigung noch mit Anionen mineralischer oder organischer Säuren assoziiert sein. Durch Sprühtrocknung der wässrigen Lösung eines Kalium- oder Calciumsalzes sind dann Granulate herstellbar, die annähernd 100 Gew.% Cyclohexenonoximether-Metallsalz enthalten.

Bei der Komponente b) handelt es sich um bekannte Handelsprodukte. Common names sind Glyphosate, Sulfosate und Glufosinate sowie entsprechende Handelsnamen Roundup®, Touchdown® bzw. Basta®.

Im Rahmen der vorliegenden Erfindungen werden insbesondere Mischungen bevorzugt, die als Derivat des N-Phosphonomethylglycins das als Glyphosate bekannte Isopropylammoniumsalz des N-Phosphonomethylglycins enthalten. Weitere bekannte Derivate des Phosphonomethylglycins sind das unter der Bezeichnung Sulfosate bekannte Trimethylsulfoniumsalz sowie das Ammoniumsalz. Besonders bevorzugt wird jedoch das eingangs erwähnte Produkt welches als Glyphosate bekannt ist, insbesondere dessen Ammoniumsalz.

Als Herbizid-Komponente b) hat sich bisher ein ein-, zwei- oder dreifach deprotoniertes Salz von Glyphosate bewährt, insbesondere ein Alkali-, Erdalkali-, Übergangsmetall- oder das Ammoniumsalz. Ganz besonders bevorzugt ist das Ammoniumsalz von Glyphosate.

In der Regel wird die Komponente b) in Überschuß, bis etwa zur 12fachen molaren Menge, insbesondere der etwa 5 bis 8fachen molaren Menge, bezogen auf die Menge an Komponente a) eingesetzt.

Unter den als Komponente c) dienenden aprotischen oder schwach protischen Lösungsmitteln sind unpolare, polare oder dipolare aliphatische oder aromatische Solventien geeignet, die keine oder nur eine geringe Lösewirkung gegenüber der Komponente b) zeigen. Um lagerstabile Formulierungen zu erhalten, sollte eine Löslichkeit des Wirkstoffs b) in der Öl-Phase unter 1 Gew.-% (bezogen auf die gesamte Formulierung) gegeben sein. Dies gilt gleichfalls für Komponente a), sofern die Salze der Verbindungen I verwendet werden. Bevorzugt sind Kohlenwasserstoffe wie Benzol, Alkylbenzol und Naphthalin, sowie deren ein- und mehrfach Alkyl-substituierten und/oder teilhydrierten Derivate, n- oder iso-Paraffine mit 8 bis 30 C-Atomen, aliphatische oder aromatische Ester von Mono- oder Dicarbonsäuren wie Methyloleat, Laurinsäure- und Adipinsäure-octylester und Benzoesäureester, oder naturbelassene oder modifizierte natürliche Fette und Öle wie Sojaöl, Sonnenblumenöl und Rapsölmethylester.

Aromatische Lösemittel der Benzol- und Naphathalin-Reihe wie Solvesso® 150 und Solvesso® 200 (Alkylaromate der Fa. Exxon) sind besonders bevorzugt.

Neben der Komponente c) enthalten die erfindungsgemäßen Formulierungen im allgemeinen Emulgatoren, Tenside und oberflächenaktive Hilfsmittel wie Netz- und Dispergiermittel als weitere Bestandteile.

Brauchbare Tenside, Netz- und Dispergiermittel sind beispielsweise:
1. Anionische Tenside und Dispergiermittel, vor allem
   - Seifen (Alkalimetall-, Erdalkalimetall- oder Ammoniumsalze von Fettsäuren), z. B. Kaliumstearat;
   - Alkylsulfate;
   - Alkylethersulfate, z. B. sulfatierte Hexa-, Hepta- und Octadecanole und Fettalkoholglykolether;
   - Alkyl- oder Isoalkylsulfonate;
   - Alkalimetall-, Erdalkalimetall- oder Ammoniumsalze von Arylsulfonsäuren oder Alkylbenzolsulfonsäuren, z.B. Lignin-, Phenolsulfonsäuren, Naphthalin- und Dibutylnaphthalin-sulfonsäuren oder Na-Dodecylbenzolsulfonate;
   - Alkylnaphthalinsulfonate;
   - Alkylmethylestersulfonate;
   - Acylglutamate;
   - Alkylbernsteinsäureestersulfonate;
   - Mono/Diphosphorsäurealkylester;
   - Sarkosinate, z.B. Na-Lauroylsarkosinat;
   - Taurate;
   - Kondensationsprodukte von sulfoniertem Naphthalin und seiner Derivate mit Formaldehyd;
   - Kondensationsprodukte von Naphthalinsulfonsäuren, Phenol- und/oder Phenol-sulfonsäuren, Formaldehyd und Harnstoff;
   - Eiweißhydrolysate;
   - Lignin-Sulfitablaugen und Methylcellulose, wobei diese Stoffe insbesondere als Dispergiermittel wirken;
   - Aerosol® OT-A;
2. Kationische Tenside:
   - Alkyltrimethylammonium-Halogenide/Alkylsulfate;
   - Alkylpyridinium-Halogenide;
   - Dialkyldimethylammonium-Halogenide/Alkylsulfate;
3. Nichtionische Tenside:
   - Fettsäure-Polyoxyethylenester wie Laurylalkohol-Polyoxyethylenetheracetat;
   - Alkyl-Polyoxyethylen- oder -Polyoxypropylenether etwa von iso-Tridecylalkohol und Fettalkohol-Polyoxyethylenether;
   - Alkylarylalkohol-Polyoxyethylenether wie Octylphenol-Polyoxyethylenether;
   - Alkoxylierte tierische/pflanzliche Fette oder Öle wie Maisölethoxylate, Rizinusölethoxilate und Talgfettethoxylate;
   - Glycerinester wie Glycerinmonostearat;
   - Fettalkoholalkoxylate oder Oxoalkoholalkoxylate;
   - Fettsäurealkoxylate wie Ölsäureethoxylate;
   - Alkylphenolalkoxylate wie ethoxyliertes iso-Octyl-, Octyl- oder Nonylphenol und Tributylphenol-Polyoxyethylenether;
   - Fettaminalkoxylate;
   - Fettsäureamidalkoxylate;
   - Zuckertenside, Sorbitester wie Sorbitanfettsäureester (Sorbitanmonooleat,Sorbitantristearat), Polyoxyethylensorbitanfettsäureester, Alkylpolyglycoside und N-Alkylgluconamide;
   - Alkylmethylsulfoxide;
   - Alkyldimethylphosphinoxide wie Tetradecyldimethylphosphinoxid;
   - Polyoxyethylen-Zuckeralkoholfettalkyl-carboxylate wie Polyoxyethylen-(40)-sorbitolhexaoleate Atlas G 1086 (CAS-Nr. 057171-56-9) der Fa. ICI Surfactants;
4. Zwitterionische Tenside:
   - Sulfobetaine;
   - Carboxybetaine;
   - Alkyldimethylaminoxide wie Tetradecyldimethylamminoxid;
5. Polymer-Tenside:
   - Di- Tri- und Multi-Blockpolymere vom Typ (AB)ₓ, ABA und BAB, z.B. Polyethylenoxid-block-polypropylenoxid oder Polystyrol-block-polyethylenoxid;
   - AB-Kammpolymere, z.B. Polymeth/acrylat-comb-polyethylenoxid;
6. andere Tenside, z.B.
   - Perfluortenside;
   - Silicontenside;
   - Phospholipide wie Lecithin- oder chemisch-modifizierte Lecithine;
   - Aminosäuretenside wie N-Lauroylglutamat;
   - oberflächenaktive Homo- und Copolymere wie Polyvinylpyrrolidon, Polyacrylsäure, Polyvinylalkohol, Polyethylenoxid, Maleinsäureanhydrid-Isobuten-Copolymere und vinylpyrrolidon-Vinylacetat-Copolymere.

Auch Mischungen der vorstehend genannten Tenside kommen in Betracht.

Besonders geeignet sind die nichtionischen Tenside aus der Gruppe der Polyoxyethylen-Zuckeralkoholfettalkyl-carboxylate, z.B. Polyoxyethylen-(40)-sorbitolhexaoleate Atlas G 1086 (CAS-Nr. 057171-56-9) der Fa. ICI Surfactants, sowie ionische Tenside aus der Reihe der Alkyl-, Dialkyl- oder Alkylarylsulfonat-Alkali- und Erdalkali-Reihe. Vorteilhaft sind ferner Emulgatoren und Tenside, die durch Umsetzung eines natürlichen Öls, insbesondere Rizinusöl, mit Ethylen- oder Propylenoxid erhalten wurden (vgl. hierzu z.B. die Ausführungen in der DE-A 19 701 123).

Die Alkylketten der o.g. Tenside, Netz- und Dispergiermittel können dabei linear oder verzweigt sein, wobei die Alkylkettenlängen im allgemeinen bei C₈ bis C₂₀ liegen.

Die erfindungsgemäßen Formulierungen können des weiteren - zur Verbesserung der physikalischen Eigenschaften hinsichtlich geringerer Serumbildung oder geringerer Sedimentation - sogenannte Verdickungsmittel enthalten, worunter allgemein mineralische Bestandteile wie Bentonite, Talicite und Hektorite oder Rizinusöl-Derivate zu verstehen sind. Aufgrund der resultierenden Viskositätserhöhung werden gegebenenfalls chemische Prozesse in der Formulierung bei deren Lagerung unterdrückt, was dann zu verbesserter Wirkstoff-Stabilität führen kann.

Bezüglich wirkungssteigernder Hilfsmittel - wie Adipinsäureester, Methyloelat und anderer technischer Ester, basierend auf natürlich vorkommenden Carbonsäuren, Dicarbonsäuren oder Fettsäuren - sei auf die WO 96/22020, die DE-A 44 45 546 sowie die dort zitierte Literatur verwiesen.

Alle genannten Hilfsmittel können vor oder nach der Vermahlung dem Formulieransatz zugegeben werden. Ihr Gesamt-Anteil an der Formulierung beträgt in der Regel 0 bis 80 Gew.%, insbesondere 5 bis 40 Gew.%.

Die Formulierungen können gewünschtenfalls auch 0 bis 60 Gew.%, insbesondere 1 bis 30 Gew.%, eines dritten herbiziden Wirkstoffs aus der Gruppe der Aryloxyphenoxypropionsäuren und ihrer Ester enthalten, vorzugsweise Clodinafop, Cyhalofop, Fenoxaprop, Fluazifop, Haloxyfop, Propaquizafop, Quizalofop oder einen Ester dieser Verbindungen, insbesondere Clodinafop, Quizalofop, Quizalofop-ethyl oder Quizalofop-tefuryl, enthalten.
Auch die Enantiomeren dieser Verbindungen wie Quizalofop-P, Quizalofop-P-ethyl und Quizalofop-P-tefuryl kommen in Betracht.

Die erfindungsgemäßen neuen Formulierungen werden vorteilhaft erst mit Wasser verdünnt, z.B. im Tankmixverfahren, bevor sie im Vorauflauf- oder Nachauflaufverfahren auf die unerwünschten Pflanzen oder deren Lebensraum appliziert werden. Die Menge an Wasser beträgt dabei z.B. 100 bis 400 l/ha.

Zur Tankmix-pH-Absenkung und zur weiteren Wirkungssteigerung kann es auch vorteilhaft sein, übliche Tankmix-Adjuvantien, in einer Menge von 0,1 bis 5,0 kg/ha bzw. 0,1 bis 5,0 l/ha zuzusetzen, z.B. Ammonium-Salze wie Ammomiumsulfat und Ammoniumnitrat, Harnstoff, Öl-Emulgator-Zusätze und insbesondere Dash HC (von BASF).

Die erfindungsgemäßen Suspensionskonzentrate werden dadurch hergestellt, daß man die Wirkstoffe a) und b) in kristalliner Form und die Komponente c) sowie gewünschtenfalls Hilfsmittel und/oder weitere herbizide Wirkstoffe mit herkömmlichen Kugel-, Perl- oder Rührwerksmühlen intensiv vermahlt.

Als Mahlkörper verwendet man dabei beispielsweise Glasmahlkörper oder andere mineralische oder metallische Mahlkörper in einer Größe von 0,1 bis 30 mm, vorzugsweise 0,6 bis 2 mm, und zerkleinert die Suspensionen in der Regel solange, bis die mittlere Partikelgröße deutlich unter 10 µm liegt.

Besonders vorteilhaft ist dabei, daß sich die feinpartikulären Wirkstoffe in ihrer Salzform bei Verdünnung mit Wasser im Tankmix quantitativ auflösen. Dadurch steht der Pflanze der Wirkstoff homogen und praktisch monomolekular zur Verfügung, wodurch allgemein besonders günstige herbizide Eigenschaften erreicht werden.

Hohe Anteile an lipophilen Hilfsmitteln und bipolaren Tensiden unterstützen dabei vorteilhaft die Penetration oder Transduktion/Transmission der Wirkstoffe in den Blättern. Derartige Hilfsmittel sind native Fette und Öle und insbesondere deren FettsäureMethylester, z. B. Methyloleat.

Durch die vorliegenden, weitgehend wasserfreien Formulierungen wird ferner eine Applikation im ULV-Verfahren (ultra light application) ermöglicht, wobei z. B. zur Flugzeug-Applikation die Formulierung direkt mit einem wasserfreien Öl-Konzentrat (z.B. Spraytex-Öl, ein Produkt der Fa. Exxon) von ca. 10-50 l pro Hektar mit allgemein guter Kompatibilität vermischt bzw. verdünnt werden kann.

Die erfindungsgemäßen Formulierungen zeichnen sich durch eine hervorragende Wirkung gegen ein breites Spektrum von unerwünschten Schadpflanzen aus.

Daneben eignen sie sich auch besonders bevorzugt zum Einsatz für die Bekämpfung von unerwünschtem Pflanzenwuchs in Kulturen, in denen die Kulturpflanze durch geeignete Modifikation gegen das an sich als Totalherbizid wirkende N-Phosphonomethylglycin mit einer erhöhten Resistenz versehen wurden.

Die erfindungsgemäßen Formulierungen werden als Herbizide verwendet. Die entsprechenden herbiziden Mittel bekämpfen Pflanzenwuchs auf Nichtkulturflächen sehr gut, besonders bei hohen Aufwandmengen. In Gen-modifizierten dikotylen Kulturen mit erhöhter Resistenz gegen Glyphosate oder Gluphosinate wie Soja, Raps, Zukkerrübe, Flachs, Erbse, Kartoffel, Linse und Baumwolle wirken sie gegen Unkräuter und Schadgräser, ohne die Kulturpflanzen nennenswert zu schädigen. Dieser Effekt tritt bereits bei niedrigen Aufwandmengen auf.

Besonders eignen sich die erfindungsgemäßen Formulierungen für die Anwendung zur Bekämpfung von unerwünschtem Pflanzenwuchs in Sojakulturen, wobei die Sojapflanzen selbst eine Resistenz gegen N-Phosphonomethylglycin und dessen Ester oder Salze aufweisen. Als vorteilhaft hat sich hierbei insbesondere herausgestellt, daß selektiv unerwünschte Maispflanzen bekämpft werden können, die in Sojakulturen infolge des jährlichen Fruchtwechsels zwischen Soja und Mais auftreten können (sog. "volunteer corn").

In Abhängigkeit von der jeweiligen Applikationsmethode können die erfindungsgemäßen Formulierungen bzw. die aus ihnen hergestellten Spritzbrühen noch in einer weiteren Zahl von modifizierten Kulturpflanzen zur Beseitigung unerwünschter Pflanzen eingesetzt werden. In Betracht kommen beispielsweise folgende Kulturen: Allium cepa, Ananas comosus, Arachis hypogaea, Asparagus officinalis, Beta vulgaris spec., altissima, Beta vulgaris spec., rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Um eine Spritzbrühe zur Bekämpfung von unewünschten Pflanzen herzustellen kann die Formulierung mit Ammoniumsalzen, Wasser und gewünschtenfall weiteren Tankmix-Adjuvantien gemischt werden.

Die Applikation der Mischungen bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen der fertigen Spritzbrühe beträgt je nach Bekämpfungsziel, Jahreszeit, Zielpflanzen und Wachstumsstadium 0,001 bis 3,0, vorzugsweise 0,01 bis 2,0 kg/ha an aktiven Substanzen (a.S.).

Außerdem kann es von Nutzen sein, die fertige Spritzbrühe allein in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Bekämpfung von unerwünschtem Pflanzenwuchs erfolgt so, daß man eine herbizid wirksame Menge einer Pflanzenschutzwirkstoff-Formulierung basierend auf der erfindungsgemäßen Formulierung auf die Kulturpflanze, deren Lebensraum und/oder auf deren Saatgut einwirken läßt.

### Herstellungsbeispiele

### Beispiel 1

Unter Verwendung von 0,9-1,2 mm großen Glasperlen als Mahlhilfsmittel wurden allgemein bei ca. 0-30°C die Wirkstoffe der Komponenten a) und b) mit der Komponente c) und gewünschtenfalls weiteren Formulierungs-Hilfsstoffen und/oder herbiziden Wirkstoffen vermahlen.

Wurden die Cyclohexenonoximether I als freie Säuren eingesetzt, so empfahl sich deren Zugabe erst nach der Vermahlung von Komponente b).

Die Wirkstoffkonzentration betrug insgesamt ca. 10-60 Gew.-%, allgemein 30-60 Gew.-%.
Die Mahlungen erfolgten in einer Dynomühle der Fa. Bachofen mit einer Ansatzgröße von 0,5 bis zu einem Liter in sogenannter Passagenfahrweise. Nach in der Regel 5 Passagen (Durchpumpen des Slurry durch die Mühle mit Hilfe einer Schlauchpumpe) wurden dabei nach mikroskopischer Auswertung mittlere Teilchengrößen von 1-10 µm erreicht.
Anschließend erfolgte die Einarbeitung und Verdünnung mit weiteren Hilfsmitteln und gegebenenfalls eines Wirkstoffs I (als freie Säure) durch 10 Min. Homogenisieren mit KPG- oder Magnetrührern.

### Beispiel 2

### Einsatzstoffe (allgemeine Grundrezeptur):

240-550 g/l Glyphosate (berechnet auf reinen Wirkstoffanteil)
10-80 g/l Cyclohexenonoximether
100-350 g/l Emulgator(en)
ad 1 l aprotische Verdünnungs-/Lösemittel

Pro 1 Öl-SC wurde gemäß o.g. Grundrezeptur Glyphosat - als freie Verbindung oder als Salz - zusammen mit den Emulgatoren auf 0,9 l Volumen berechnet angesetzt:

Die Komponenten wurden durch Rühren 1-2 Minuten vorhomogenisiert und per Schlauchpumpe mit einer Glasperl- bzw. Dynomühle vermahlen.

Je nach Beschaffenheit bzw. je nach Kristallgröße des Wirkstoffs Glyphosate mußte ggf. vorher der trockene Wirkstoff vorzerkleinert werden, z.B. mit einer Stiftmühle.

### Mahlparameter:

Mahlbehältervolumen 0,5 l, Glasperlschüttung ca. 80 %¹⁾, Glasperlen 1,0-1,4 mm Durchmesser; 5 Passagen diskontinuierlich; Kühlung der Mühle mit Wasser (10°C Eingangs- und ca. 20°C Ausgangstemperatur).
Typische Partikelgrößen: 0,1 bis 10 µm nach der Mahlung, davon insbesondere 30-80 % < 2 µm.

Man erhielt homogene, teils leicht viskose ÖL-SCs bzw. Glyphosate-Öl-SC-Vorkonzentrate.
Anschließend wurden die vorgesehenen Mengen an Cyclohexenonoximether I - ggf. als verdünnte Vorkonzentrate - bei ca. 20°C zum Glyphosate-Öl-SC eingerührt (ca. 30 Min. mittels Dissolver bei 800 U/min). Schließlich wurde die Mischung mit Löse- bzw. Verdünnungsmittel auf 1,0 l aufgefüllt.

Setzte man alle Wirkstoffe (d.h. I und Glyphosate/Gluphosinate) in Salz-Form ein, so kann es vorteilhaft sein, zunächst getrennte Öl-Suspensionskonzentrate der Komponente a) und b) herzustellen (sog. Master SCs).
¹⁾ d.h. Mühle zu 80 Vol.-% mit Glasperlen gefüllt

### Beispiel 3: Lagerstabilität

Die Herstellung der Glyphosate-Salze erfolgte in an sich bekannter Weise durch Vermischen bzw. homogenisieren der korrespondierenden Metall-hydroxide bzw. -carbonate und Glyphosate, eindampfen im Vakuum und trocknen im Vakuumtrockenschrank bei 1000-5000Pa (10-50 mbar) über Nacht, wonach die Restfeuchten (Wassergehalt) unter 0,5 % lagen.
Die Mengenangaben für Glyphosate-Salze sind auf reinen Wirkstoff Glyphosate berechnet.

Die Angaben zur Stabilität erfolgen nur für den Cyclohexenonoximether, da Glyphosate und seine Salze in den erfindungsgemäßen Mischungen im allgemeinen keinen Wirkstoffabbau erfuhren.

Als Cyclohexenonoximether I wurde 2-[1-(3-Chlorallyloxy)-iminopropyl]-5-(tetrahydropyran-4-yl)-3-hydroxy-cyclohex-2-enon verwendet.

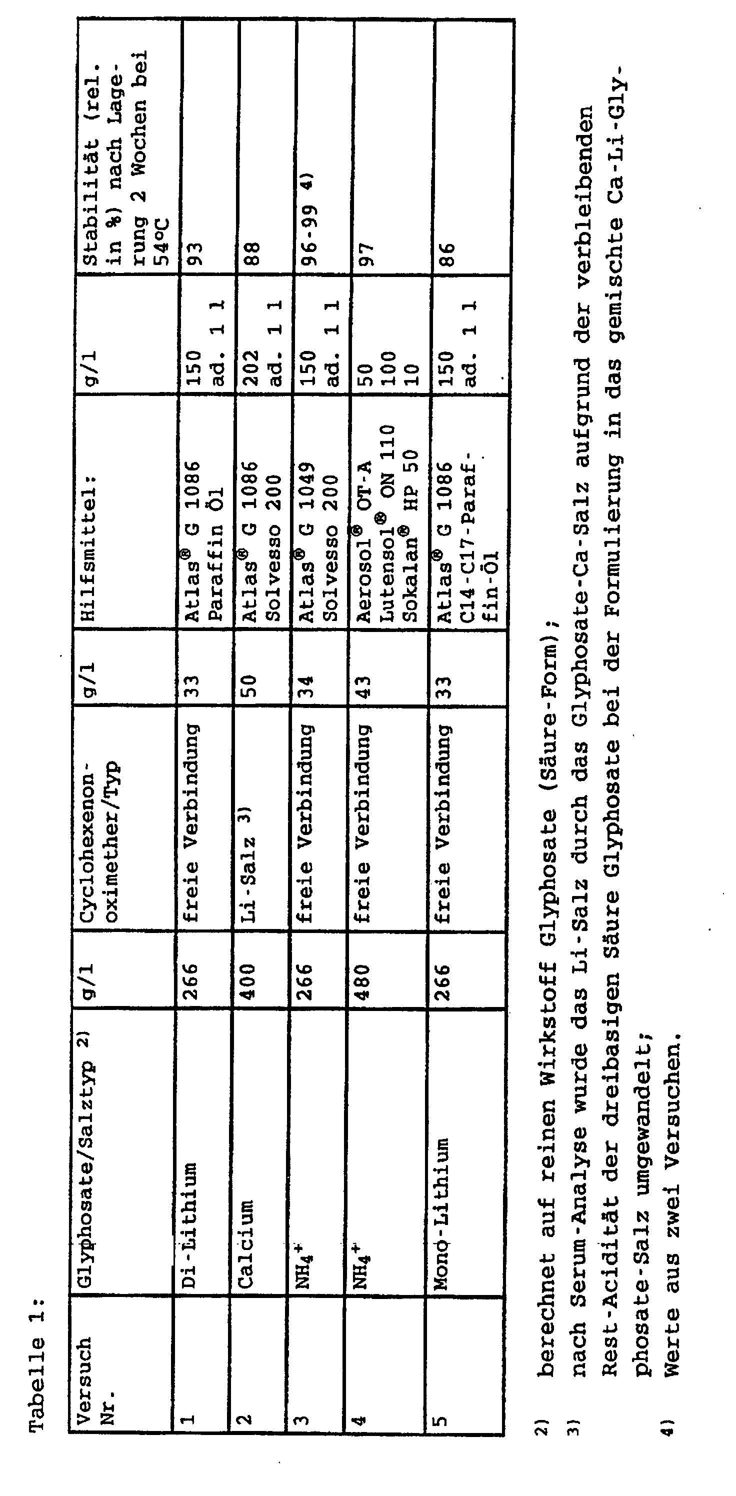

### Erläuterungen zu den Hilfsmitteln:

Atlas G 1086 ist ein Polyoxyethylen-(40)-sorbitolhexaoleat (CAS-Nr. 057171-56-9); Produkt der Fa. Uniqema, vormals ICI Surfactants;
Solvesso 200 ist ein Alkyl-substituierter C10-Aromat; Produkt der Fa. Exxon;
Aerosol OT-A ist ein Natriumdioctyl-sulfosuccinat (CAS-Nr. 000577-11-7); Produkt der Fa. Cytec;
Lutensol ON 110 ist Isodecanol ethoxyliert; Produkt der Fa. BASF AG;
Sokalan HP 50 ist ein Polyvinylpyrrolidon; Produkt der Fa. BASF AG;

### Beispiel 4:

### Vergleichsversuche

Bei den Vergleichsversuchen wurde 2-[1-(3-Chlorallyloxy)-iminopropyl]-5-(tetrahydropyran-4-yl)-3-hydroxy-cyclohex-2-enon ⁵⁾ mit den in Tabelle 2 angegebenen Hilfsmitteln durch Rühren in die Handelsware Roundup® Ultra (Produkt und Label der Fa. Monsanto) eingearbeitet. Der Glyphosate-Gehalt der erhaltenen Zubereitungen lag dann jeweils bei 25 Gew.-%. Nach 2 Wochen Lagerung prüfte man den noch vorhandenen Anteil des o.g. Cyclohexenonoximethers.

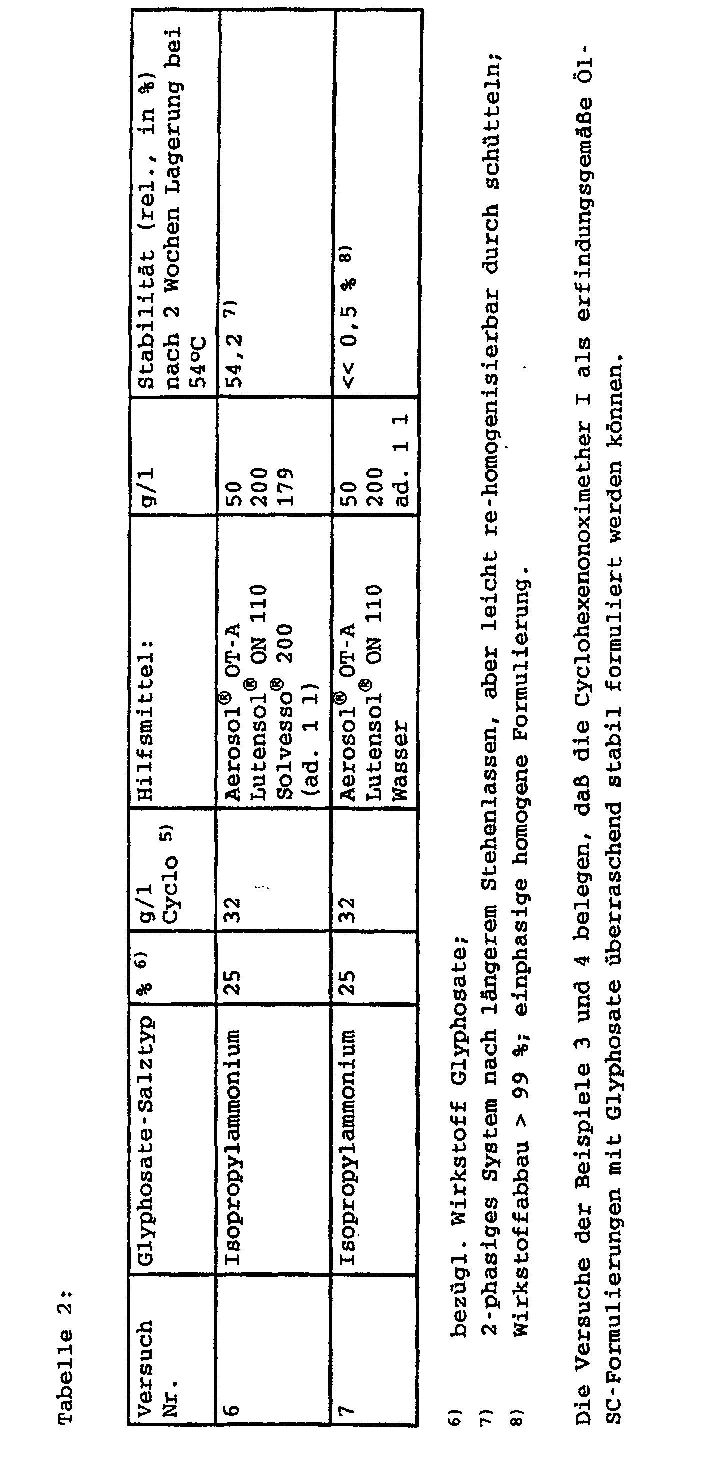

## Patentansprüche

1. Weitgehend wasserfreie Formulierung von Pflanzenschutzwirkstoffen, bestehend im Wesentlichen aus
a) wenigstens einem Cyclohexenonoximether der Formel I 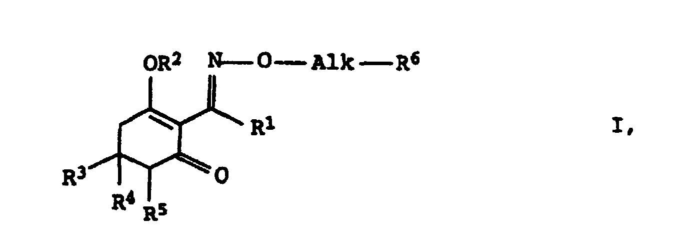 wobei die Variablen folgende Bedeutungen haben:
R¹ Ethyl oder Propyl;
R² Wasserstoff oder ein Äquivalent eines landwirtschaftlich brauchbaren Kations;
R³ 2-(Thioethyl)propyl, Tetrahydrothiopyran-3-yl, Tetrahydrothiopyran-4-yl, Tetrahydropyran-3-yl, Tetrahydropyran-4-yl, 1-(Methylthio)cyclopropyl, 5-(Isopropyl)isoxazol-3-yl, 2,5-Dimethylpyrazol-3-yl, 2,4,6-Trimethylphenyl oder 2,4,6-Trimethyl-3-butyrylphenyl;
R⁴ und R⁵ unabhängig voneinander Wasserstoff, Methyl oder Methoxycarbonyl;
Alk CH₂CH₂, CH₂CH(CH₃), CH₂CH=CH, CH₂CH=C(Cl) oder CH₂CH₂CH=CH;
R⁶ Wasserstoff, Phenyl, Halogenphenyl, Dihalogenphenyl, Phenoxy, Halogenphenoxy oder Dihalogenphenoxy;
b) N-Phosphonomethylglycin, ein Ester oder Salz hiervon, DL-Homoalanin-4-yl(methyl)phosphinsäure oder dessen Ammoniumsalz;
c) 20 bis 80 Gew.% eines aprotischen oder schwach protischen Lösungsmittels, in dem die Komponenten a) und b) gelöst oder suspendiert sind;
d) gewünschtenfalls Emulgatoren, Tenside, oberflächenaktive und/oder wirkungssteigernde Hilfsmittel.

2. Weitgehend wasserfreie Formulierung nach Anspruch 1, enthaltend als Komponente a) einen Cyclohexenonoximether aus der Gruppe: Sethoxydim, Cycloxydim, Clethodim, Tralkoxydim, Butroxydim, 2-[1-(3-Chlorallyloxy)-iminopropyl]-5-(tetrahydropyran-4-yl)-3-hydroxy-cyclohex-2-enon, 2-[1-(2-p-chlorphenoxypropyloxy)-iminobutyl]-5-(tetrahydrothiopyran-3-yl)-3-hydroxy-cyclohex-2-enon, deren Alkali- oder Erdalkalimetallsalze oder Gemische dieser Wirkstoffe.

3. Weitgehend wasserfreie Formulierung nach Anspruch 1, enthaltend als Komponente b) ein ein-, zwei- oder dreifach deprotoniertes Salz von Glyphosate.

4. Weitgehend wasserfreie Formulierung nach Anspruch 1 oder 2, enthaltend zusätzlich 0 bis 80 Gew.% mindestens eines Formulierungs-Hilfsstoffs, ausgewählt aus den Klassen der grenzflächenaktiven ionischen oder nichtionischen Tenside, Dispergatoren, anderer Lösungsmittel und Verdickungsmittel.

5. Weitgehend wasserfreie Formulierung nach Anspruch 1, enthaltend zusätzlich 0 bis 60 Gew.% eines dritten herbiziden Wirkstoffs aus der Gruppe der Aryloxyphenoxypropionsäuren und ihrer Ester.

6. Verfahren zur Herstellung einer Spritzbrühe zur Bekämpfung von unerwünschten Pflanzen, **dadurch gekennzeichnet, daß** man eine Formulierung gemäß Anspruch 1 mit Ammoniumsalzen, Wasser und gewünschtenfalls weiteren Tankmix-Adjuvantien mischt.

7. 2-[1-(3-Chlorallyloxy)-iminopropyl]-5-(tetrahydropyran-4-yl)-3-hydroxy-cyclohex-2-enon-Lithiumsalz.

8. Öl-Suspensionskonzentrat, bestehend im Wesentlichen aus
a) wenigstens einem Cyclohexenonoximether der Formel I 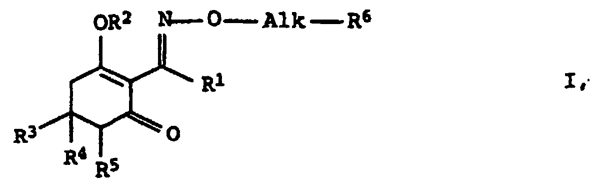 wobei die Variablen folgende Bedeutungen haben:
R¹ Ethyl oder Propyl;
R² ein Äquivalent eines landwirtschaftlich brauchbaren Kations;
R³ 2-(Thioethyl)propyl, Tetrahydrothiopyran-3-yl, Tetrahydrothiopyran-4-yl, Tetrahydropyran-3-yl, Tetrahydropyran-4-yl, 1-(Methylthio)cyclopropyl, 5-(Isopropyl)isoxazol-3-yl, 2,5-Dimethylpyrazol-3-yl, 2,4,6-Trimethylphenyl oder 2,4,6-Trimethyl-3-butyrylphenyl;
R⁴ und R⁵ unabhängig voneinander Wasserstoff, Methyl oder Methoxycarbonyl;
Alk CH₂CH₂, CH₂CH(CH₃), CH₂CH=CH, CH₂CH=C(Cl) oder CH₂CH₂CH=CH;
R⁶ Wasserstoff, Phenyl, Halogenphenyl, Dihalogenphenyl, Phenoxy, Halogenphenoxy oder Dihalogenphenoxy;
c) 20 bis 80 Gew.% eines aprotischen oder schwach protischen Lösungsmittels, in dem die Komponente a) suspendiert ist;
d) gewünschtenfalls Emulgatoren, Tenside, oberflächenaktive und/oder wirkungssteigernde Hilfsmittel.

9. Öl-Suspensionskonzentrat nach Anspruch 8, enthaltend als Komponente a) wenigstens ein Cyclohexenonoximether-Salz der Formel I, worin R² ein Äquivalent eines Alkali- oder Erdalkalimetallkations bedeutet.

10. Öl-Suspensionskonzentrat nach Anspruch 9, wobei das Cyclohexenonoximether-Salz in der Ölphase zu weniger als 1% gelöst vorliegt.

## Claims

1. A substantially water-free formulation of crop protection agents, comprising essentially
a) at least one cyclohexenone oxime ether of the formula I 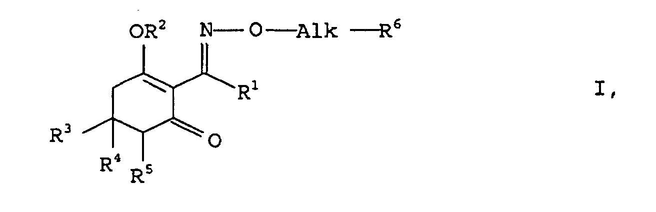 where the variables are defined as follows:
R¹ is ethyl or propyl;
R² is hydrogen or an equivalent of an agriculturally useful cation;
R³ is 2-(thioethyl)propyl, tetrahydrothiopyran-3-yl, tetrahydrothiopyran-4-yl, tetrahydropyran-3-yl, tetrahydropyran-4-yl, 1-(methylthio)cyclopropyl, 5-(isopropyl)isoxazol-3-yl, 2,5-dimethylpyrazol-3-yl, 2,4,6-trimethylphenyl or 2,4,6-trimethyl-3-butyrylphenyl;
R⁴ and R⁵ independently of one another are each hydrogen, methyl or methoxycarbonyl;
Alk is CH₂CH₂, CH₂CH(CH₃), CH₂CH=CH, CH₂CH=C(Cl) or CH₂CH₂CH=CH;
R⁶ is hydrogen, phenyl, halophenyl, dihalophenyl, phenoxy, halophenoxy or dihalophenoxy;
b) N-phosphonomethylglycine, an ester or salt thereof, DL-homoalanin-4-yl(methyl)phosphinic acid or its ammonium salt;
c) from 20 to 80 % by weight of an aprotic or weakly protic solvent in which the components a) and b) are dissolved or suspended;
d) if desired emulsifiers, surfactants, surface-active and/or activity-enhancing auxiliaries.

2. A substantially water-free formulation according to claim 1, comprising as component a) a cyclohexenone oxime ether selected from the group consisting of: sethoxydim, cycloxydim, clethodim, tralkoxydim, butroxydim, 2-[1-(3-chloroallyloxy)iminopropyl]-5-(tetrahydropyran-4-yl)-3-hydroxycyclohex-2-enone, 2-[1-(2-p-chlorophenoxypropyloxy)iminobutyl]-5-(tetrahydrothiopyran-3-yl)-3-hydroxycyclohex-2-enone, their alkali metal or alkaline earth metal salts or mixtures of these active ingredients.

3. A substantially water-free formulation according to claim 1, comprising as component b) a mono-, di- or trideprotonated salt of glyphosate.

4. A substantially water-free formulation according to claim 1, comprising additionally from 0 to 80 % by weight of at least one formulation auxiliary selected from the classes of the surface-active ionic or nonionic surfactants, dispersants, other solvents and thickeners.

5. A substantially water-free formulation according to claim 1, comprising additionally from 0 to 60 % by weight of a third herbicidally active compound selected from the group consisting of the aryloxyphenoxypropionic acids and their esters.

6. A process for preparing a spray liquor for controlling undesirable plants, which comprises mixing a formulation as claimed in claim 1 with ammonium salts, water and, if desired, other tankmix adjuvants.

7. 2-[1-(3-chloroallyloxy)iminopropyl]-5-(tetrahydropyran-4-yl)-3-hydroxycyclohex-2-enone lithium salt.

8. An oil suspension concentrate, comprising essentially
a) at least one cyclohexenone oxime ether salt of the formula I 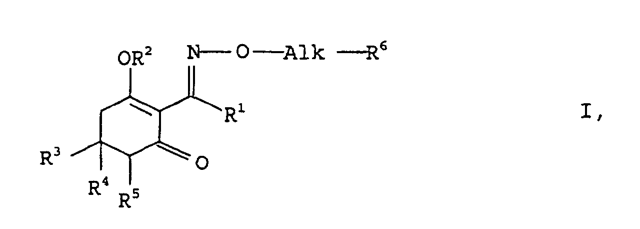 where the variables are defined as follows:
R¹ is ethyl or propyl;
R² is an equivalent of an agriculturally useful cation;
R³ is 2-(thioethyl)propyl, tetrahydrothiopyran-3-yl, tetrahydrothiopyran-4-yl, tetrahydropyran-3-yl, tetrahydropyran-4-yl, 1-(methylthio)cyclopropyl, 5-(isopropyl)isoxazol-3-yl, 2,5-dimethylpyrazol-3-yl, 2,4,6-trimethylphenyl or 2,4,6-trimethyl-3-butyrylphenyl;
R⁴ and R⁵ independently of one another are each hydrogen, methyl or methoxycarbonyl;
Alk is CH₂CH₂, CH₂CH(CH₃), CH₂CH=CH, CH₂CH=C(Cl) or CH₂CH₂CH=CH;
R⁶ is hydrogen, phenyl, halophenyl, dihalophenyl, phenoxy, halophenoxy or dihalophenoxy;
c) from 20 to 80 % by weight of an aprotic or weakly protic solvent in which the component a) is suspended;
d) if desired emulsifiers, surfactants, surface-active and/or activity-enhancing auxiliaries.

9. The oil suspension concentrate according to claim 8, comprising as component a) at least one cyclohexenone oxime ether salt of formula I, wherein R² is an equivalent of an alkali metal or alkaline earth metal cation.

10. The oil suspension concentrate according to claim 9, comprising as component a) at least one cyclohexenone oxime ether salt is present in solution in the oil phase in an amount of less than 1%.

## Revendications

1. Formulation en grande partie anhydre de matières phytoprotectrices se composant essentiellement de
a) au moins un cyclohexènone-oxime-éther de formule 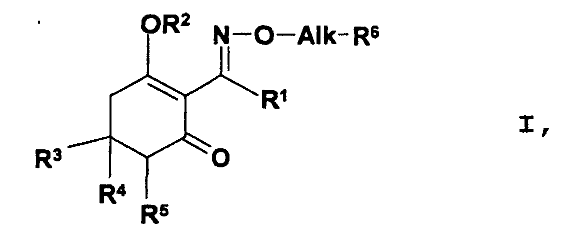 dans laquelle les variables prennent les significations suivantes :
R¹ représente un groupe éthyle ou propyle;
R² représente un atome d'hydrogène ou un équivalent d'un cation que l'on peut employer en agriculture;
R³ représente un groupe 2-(thioéthyl)propyle, tétrahydrothiopyran-3-yle, tétrahydrothiopyran-4-yle, tétrahydropyran-3-yle, tétrahydropyran-4-yle, 1-(méthylthio)cyclopropyle, 5-(isopropyl)isoxazol-3-yle, 2,5-diméthylpyrazol-3-yle, 2,4,6-triméthyl-phényle, ou 2,4,6-triméthyl-3-butyrylphényle;
R⁴ et R⁵ représentent indépendamment l'un de l'autre, un atome d'hydrogène, un groupe méthyle ou méthoxycarbonyle;
Alk représente CH₂CH₂, CH₂CH(CH₃), CH₂CH=CH, CH₂CH=C(Cl) ou CH₂CH₂CH=CH;
R⁶ représente un atome d'hydrogène, un groupe phényle, halogénophényle, dihalogénophényle, phénoxy, halogénophénoxy, ou dihalogénophénoxy;
b ) de N-phosphonométhylglycine, un ester ou sel de celle-ci, d'acide DL-homoalanine-4-yl(méthyl)phosphinique ou son sel d'ammonium;
c) 20 à 80% en poids d'un solvant aprotique ou faiblement protique, dans lequel les composants a) et b) sont dissous ou suspendus;
d) si souhaité, des émulsifiants, des tensio-actifs, des adjuvants tensio-actifs et/ou augmentant le rendement.

2. Formulation en grande partie anhydre selon la revendication 1, contenant en tant que composant a), un cyclohexénone-oxime-éther choisi dans le groupe : séthoxydim, cycloxydim, cléthodim, tralcoxydim, butroxydim, 2-[1-(3-chloroallyloxy)-iminopropyl]-5-(tétrahydro-pyran-4-yl)-3-hydroxy-cyclohex-2-énone, 2-[1-(2-p-chlorophénoxypropyloxy)iminobutyl]-5-(tétrahydrothiopyran-3-yl)-3-hydroxy-cyclohex-2-énone, leurs sels de métal alcalin ou alcalino-terreux ou des mélanges de ces matières actives.

3. Formulation en grande partie anhydre selon la revendication 1, contenant en tant que composant b), un sel de glyphosate, déprotoné une fois, deux fois ou trois fois.

4. Formulation en grande partie anhydre selon la revendication 1 ou 2, contenant en outre 0 à 80% en poids d'au moins un adjuvant de formulation, choisi dans les classes des tensio-actifs ioniques ou non ioniques surfactifs, des dispersants, des autres solvants et des épaisissants.

5. Formulation en grande partie anhydre selon la revendication 1, contenant en outre 0 à 60% en poids d'une troisième matière active herbicide choisie dans le groupe des acides aryloxyphénoxypropioniques et leurs esters.

6. Procédé de préparation d'une bouillie de pulvérisation pour lutter contre des plantes indésirables, **caractérisé en ce que** l'on mélange une formulation selon la revendication 1, avec des sels d'ammonium, de l'eau, et si souhaité, d'autres adjuvants de mélange de cuve.

7. Sel de lithium et de 2-[1-(3-chloroallyloxy)-iminopropyl]-5-(tétrahydropyran-4-yl)-3-hydroxy-cyclohex-2-énone.

8. Concentré de suspension d'huile, constitué essentiellement de
a) au moins un cyclohexène-oxime-éther de formule I 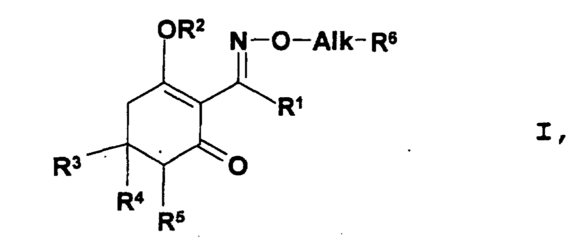 dans laquelle les variables prennent les significations suivantes :
R¹ représente un groupe éthyle ou propyle;
R² représente un équivalent d'un cation que l'on peut employer en agriculture;
R³ représente un groupe 2-(thioéthyl)propyle, tétrahydrothiopyran-3-yle, tétrahydrothiopyran-4-yle, tétrahydropyran-3-yle, tétrahydropyran-4-yle, 1-(méthylthio)cyclopropyle, 5-(isopropyl)isoxazol-3-yle, 2,5-diméthylpyrazol-3-yle, 2,4,6-triméthyl-phényle, ou 2,4,6-triméthyl-3-butyrylphényle;
R⁴ et R⁵ représentent indépendamment l'un de l'autre, un atome d'hydrogène, un groupe méthyle ou méthoxycarbonyle;
Alk représente CH₂CH₂, CH₂CH(CH₃), CH₂CH=CH, CH₂CH=C(Cl) ou CH₂CH₂CH=CH;
R⁶ représente un atome d'hydrogène, un groupe phényle, halogénophényle, dihalogénophényle, phénoxy, halogénophénoxy, ou dihalogénophénoxy;
c) 20 à 80% en poids d'un solvant aprotique ou faiblement protique, dans lequel le composant a) est en suspension;
d) si souhaité, des émulsifiants, des tensio-actifs, des adjuvants tensio-actifs et/ou augmentant l'effet.

9. Concentré de suspension d'huile selon la revendication 8, contenant comme composant a) au moins un sel de cyclohexénone-oxime-éther de formule I, dans laquelle R² désigne un équivalent d'un cation de métal alcalin ou alcalino-terreux.

10. Concentré de suspension d'huile selon la revendication 9, dans lequel le sel de cyclohexénone-oxime-éther est présent à l'état dissous à raison de moins de 1% dans la phase d'huile.
